# FASCICULE DE BREVET EUROPEEN

(11) **EP 0 648 514 B1**
(45) Date de publication et mention de la délivrance du brevet: **23.01.2002**
(21) Numéro de dépôt: 94402223.5
(22) Date de dépôt: 05.10.1994
(51) Int. Cl.: A61N 1/39

(54) **Défibrillateur/stimulateur cardiaque implantable à générateur de chocs multiphasiques**
Implantierbarer Defibrillator/Herzschrittmacher mit mehrphasigem Impulsgenerator
Implantable defibrillator/pacemaker with multiphasic shocks generator

(30) Priorité: 15.10.1993 FR 9312265
(43) Date de publication de la demande: 19.04.1995
(73) Titulaire: ELA MEDICAL, F-92541 Montrouge (FR)
(72) Inventeur: Kroiss, Daniel, F-67590 Schweighouse-Moder (FR); Ostroff, Alan, F-67250 Preuschdorf (FR); Jacobson, Peter, F-67500 Haguenau (FR)
(74) Mandataire: Laget, Jean-Loup

(56) Documents cités:
- EP-A- 0 326 290
- EP-A- 0 515 059
- EP-A- 0 540 266
- EP-A- 0 547 878
- EP-A- 0 551 746
- EP-A- 0 553 863
- EP-A- 0 553 864
- US-A- 4 800 883
- US-A- 4 998 531
- US-A- 5 224 476

## Description

L'invention concerne un circuit pour l'application de chocs à haute énergie à un défibrillateur implantable.

Dans le cas présent, le terme défibrillateur se réfère à tout dispositif pour éliminer une tachyarythmie avec une énergie électrique qui dépasse sensiblement l'énergie fournie par des stimulateurs cardiaques implantables, y compris toutes combinaisons ou sous-combinaisons de défibrillateurs, appareils de cardioversion et stimulateurs cardiaques implantables.

L'invention concerne plus particulièrement un circuit pour envoyer des chocs tronqués monophasiques ou multiphasiques à une charge à partir d'un condensateur unique, et la connexion de ce circuit au reste du défibrillateur. Dans la description :
- Un choc monophasique envoie le courant dans un sens.
- Un choc multiphasique envoie le courant d'abord dans un sens (appelé première phase du choc) puis dans le sens opposé (appelé seconde phase) et peut fournir des phases alternées additionnelles. Un choc biphasique comprend deux phases.
- Un choc tronqué met brusquement fin à l'envoi de courant dans la charge, soit en interrompant le courant dans la charge, soit en déchargeant rapidement le condensateur de stockage.
- La charge représente l'impédance des fils des sondes, des électrodes de défibrillation, de l'interface électrodes-tissus, et de la masse des tissus entre les électrodes.
- Le condensateur unique représente toute combinaison série et/ou parallèle de groupes de condensateurs qui résulte en un unique condensateur équivalent dont les deux bornes sont reliées au circuit d'application de chocs. Egalement la pile peut être composée de plusieurs piles en série ou parallèle, ou les deux.
- La stimulation est négative par rapport à sa masse. Il est d'usage dans la stimulation cardiaque de relier la masse à une électrode dite indifférente, et de relier une tension négative de stimulation à une plus petite électrode. Ceci est pour éviter la stimulation anodique, reputée être arythmogène.

Ainsi, l'invention concerne des circuits pour générer des formes d'onde multiphasiques avec un condensateur unique et ne concerne pas des circuits qui génèrent des formes d'onde multiphasiques avec des condensateurs multiples. La forme d'onde d'un condensateur unique décharge le condensateur dans la charge dans un premier sens dans la première phase, interrompt le courant, continue de décharger le condensateur dans la charge dans le sens opposé dans la seconde phase, puis tronque la décharge. En conséquence, l'amplitude du front avant de la seconde phase est égale à l'amplitude du front arrière de la première phase. La forme d'onde d'un condensateur multiple décharge un condensateur différent pour chaque sens, ou même pour chaque phase, et l'amplitude du front avant ne dépend pas nécessairement de l'amplitude du front arrière de la phase précédente.

Les anciens défibrillateurs ne fournissaient que des formes d'ondes monophasiques. Le document USRE 27652 au nom de Mirowski (priorité du 9 fév. 1970) a décrit un défibrillateur automatique à circuit d'application de chocs monophasiques qui fournissait un choc non tronqué dès que le condensateur de stockage était chargé jusqu'à une tension fixe. Le document FR 2 257 312 au nom de Zacouto (priorité du 16 janv. 1974) montrait des moyens pour appliquer des chocs monophasiques séquentiels par des paires multiples d'électrodes. Le brevet US No 4 403 614 an nom de Engle (priorité du 19 juillet 1979) et le brevet US No 4 384 585 au nom de Zipes (priorité du 6 mars 1981) ont revendiqué des moyens pour synchroniser les chocs avec les événements détectés, mais n'ont pas donné de détails concernant le circuit de décharge. Le brevet US No 4.614.192 au nom de Imran (priorité du 21 avril 1982) a ajouté des moyens pour tronquer les chocs monophasiques en déchargeant rapidement le condensateur de stockage.

Suite à des expériences concernant des chocs bidirectionnels en 1964 et 1980, J.C. Schulder et al. ont décrit un "Défibrillateur à forme d'onde bidirectionnelle à Thyratron/SCR à ultra-haute énergie", dans Med Biol Eng Comput 20:419, 1982, consistant en un générateur biphasique avec un condensateur par phase. Le document SU 1149979 au nom de Pekarski (priorité du 8 oct. 1983) a montré également un circuit d'application de chocs tronqués biphasiques avec un condensateur pour chaque phase.

En 1984, Schuder et al ont présenté les résultats d'une forme d'onde biphasique simulée tronquée à condensateur unique . Dans leur rapport intitulé "Transthoracic Defibrillation of 100 Kg Calves with Bidirectional Truncated Exponential Shocks", vol. XXX Trans Am Soc Artif Intern Organs, 1984, les auteurs ont décrit des expériences faites avec une "forme d'onde biphasée exponentielle tronquée asymétrique ... pouvant être mise en oeuvre dans un appareil de dimensions cliniques". Ils ont montré une forme d'onde où le front arrière de la première phase était égal au front avant de la seconde phase.

L'approche à condensateur unique simplifie à la fois les circuits de charge et de décharge, réduisant les dimensions, le poids et le défaut de fiabilité dans les dispositifs implantables. A mesure que les données se sont accumulées, et ont montré les résultats améliorés obtenus sur des animaux et en clinique avec des chocs tronqués biphasiques par comparaison avec des chocs monophasiques, les inventeurs ont proposé une variété de générateurs de formes d'ondes tronquées multiphasiques à condensateur unique. Tous ces circuits comprennent au moins quatre commutateurs dans une configuration de pont en H.

Les concepteurs utilisent fréquemment la configuration de pont en H pour commander du courant dans une charge dans deux directions à partir d'une source de courant continu, par exemple pour commander un moteur pas-à-pas ou un servomoteur à partir d'une pile. Pendant la première phase, un premier commutateur relie le pôle positif de la source à un premier côté de la charge et un second commutateur relie le pôle négatif de la source au second côté de la charge. Pendant la seconde phase, un troisième commutateur relie le pôle positif de la source au second côté de la charge, et un quatrième commutateur relie le pôle négatif de la source au premier côté de la charge. Les premier et troisième commutateurs, reliés au pôle positif de la source, sont appelés commutateurs du côté supérieur. Les second et quatrième commutateurs, reliés au pôle négatif de la source, sont appelés commutateurs du côté inférieur.

Les circuits à décharge implantables de l'art antérieur utilisent un ou plusieurs de trois types de commutateurs dans le pont en H. Chaque type de commutateur comprend une borne d'entrée, une borne de sortie et une borne de commande, et répond à un signal de commande entre les bornes de commande et de sortie. Les redresseurs commandés à silicium ou thyristors (SCR) deviennent conducteurs en réponse à une impulsion à la borne de commande, et ne cessent de conduire que lorsque le courant qui les traverse tombe sensiblement à zéro. Les transistors à effet de champ à métal-oxyde-semiconducteur (MOSFET) et les transistors bipolaires à porte isolée (IGBT) restent passants tant qu'une tension de commande apparaît à la borne de commande.

Selon la façon dont ils protègent les circuits de stimulation et de détection des impulsions de défibrillation, les circuits de l'art antérieur soit isolent le condensateur et le circuit de décharge de la masse de stimulation et de détection, soit relient le côté négatif du condensateur à la masse. Dans la version isolée, ils doivent fournir des signaux isolés de commande des commutateurs. Dans la version à masse négative, ils doivent toujours appliquer des signaux de commande isolés aux commutateurs du côté supérieur.

Ainsi, tout circuit d'application de choc biphasique à condensateur unique a besoin de: deux commutateurs du côté supérieur et deux commutateurs du côté inférieur reliés en un pont en H, et au moins deux dispositifs de commande de commutateurs isolés. Les brevets de l'art antérieur qui suivent décrivent tous un pont en H pour générer une forme d'onde multiphasique à condensateur unique, où la structure des moyens de commutation et des moyens de commande des commutateurs diffère dans chaque conception:

Le brevet US No 4 800 883 au nom de Winstrom (priorité du 2 avril 1986) a proposé un circuit de décharge isolé avec quatre commutateurs MOSFET et a utilisé un transformateur avec une porteuse radiofréquence (RF), un redressement et des circuits de coupure rapide pour les dispositifs de commande du côté supérieur et du côté inférieur. Un unique transformateur à deux secondaires alimentait à la fois les commutateurs du côté supérieur et du côté inférieur dans la même phase. Toutes les revendications se rapportaient à un condensateur à niveaux multiples avec des prises de tension.

Le document EP 0281219 au nom de Mehra (priorité du 14 janvier 1987) proposait un circuit de décharge à masse négative avec un SCR en série avec un MOSFET pour chaque commutateur du côté supérieur et un SCR pour chaque commutateur du côté inférieur. Mehra ne donne pas de détails concernant les dispositifs de commande des commutateurs.

Le document EP 0280526 au nom de Baker (priorité du 27 fév. 1987) utilisait le circuit de Winstrom ci-dessus, avec en plus la nécessité d'une durée de la première phase plus longue que la durée de la seconde phase (à noter qu'en 1984, Jones et al ont publié des résultats pour des impulsions de défibrillation avec une première phase de 5 ms et une seconde phase de 1 ms, voir Am. J. Physiol. 247 (Heart Circ. Physiol. 16).

Le document EP 0324380 au nom de Bach (priorité du 12 janv. 1988) a proposé un autre circuit de décharge à masse négative avec des SCR pour les commutateurs du côté supérieur et des MOSFET pour les commutateurs du côté inférieur. Bach utilisait des transformateurs d'impulsions pour les dispositifs de commande du côté supérieur et commandait directement ceux du côté inférieur. Le document EP 0326290 au nom de de Coriolis (priorité du 19 janv. 1988) a divulgué un appareil selon le préambule de la revendication 1. Ce document proposait un autre circuit de décharge à masse négative avec deux SCR en série pour le commutateur du côté supérieur de la première phase, un MOSFET pour le commutateur du côté inférieur de la première phase, et des SCR pour les commutateurs du côté supérieur et du côté inférieur de la seconde phase. De Coriolis tronquait la seconde phase par une décharge rapide du condensateur de stockage par le commutateur du côté supérieur de la première phase et le commutateur du côté inférieur de la seconde phase. De Coriolis commandait les commutateurs du côté supérieur avec des transformateurs d'impulsions et les commutateurs du côté inférieur avec des décaleurs de niveau raccordés à une alimentation positive.

Le brevet US No 4 998 531 au nom de Bocchi (priorité du 28 mars 1990) proposait un autre circuit de décharge à masse négative avec quatre commutateurs MOSFET. Bocchi utilisait des décaleurs de niveau pour les dispositifs de commande du côté inférieur et utilisait un transformateur pour chaque dispositif de commande du côté supérieur où une impulsion dans un sens rendait passant le MOSFET et une impulsion dans le sens inverse le bloquait.

Le brevet US No 5 111 816 au nom de Pless (priorité du 22 oct. 1990) proposait un autre circuit de décharge à masse négative avec des commutateurs IGBT et MOSFET. Toutes les variantes de Pless commandaient à la fois les commutateurs du côté supérieur et du côté inférieur dans la même phase à partir d'un transformateur commun avec une porteuse RF et un redressement, et un circuit de coupure rapide pour au moins un commutateur de chaque phase. Pless raccordait également la borne négative de la pile à la masse et l'inversait pour établir la tension de stimulation.

Il y a cependant possibilité d'améliorer les générateurs de chocs tronqués multiphasiques à condensateur unique.

Toutes les conceptions de l'art antérieur soit isolent le circuit de décharge de la masse de stimulation, soit relient le pôle négatif du condensateur de stockage à la masse de stimulation. Ceci fait qu'il est nécessaire d'établir une alimentation positive pour les dispositifs de commande des commutateurs du côté inférieur et une alimentation négative pour la stimulation. Il est également nécessaire d'alimenter le circuit de commande soit par l'alimentation positive soit par l'alimentation négative, puis d'inverser les signaux de commande soit pour la stimulation, soit pour la commande des chocs.

Un but de l'invention est donc de simplifier le circuit d'alimentation et le circuit de commande du circuit de décharge dans un défibrillateur /stimulateur cardiaque implantable.

Un autre but de l'invention est de faire fonctionner la stimulation, la détection, la logique de commande et la commande des commutateurs de chocs du côté inférieur, sur toute une gamme de tensions négatives par rapport à la masse de stimulation.

Un autre but encore de l'invention est de dériver toutes les alimentations destinées à la stimulation, la détection, la logique de commande et la commande des commutateurs de chocs du côté inférieur, à partir d'une alimentation par pile sans inversion. De telles configurations à polarité unique sont plus faciles à réaliser dans un circuit intégré.

Pour atteindre ces buts, l'invention relie la borne positive de la pile à la masse, et relie le côté inférieur du circuit de décharge à pont en H et du condensateur de stockage à une tension négative par rapport à la masse.

L'invention fait fonctionner le circuit de commande entre une première tension négative et la masse, le circuit de stimulation entre une seconde tension négative et la masse, et la commande des commutateurs de choc du côté inférieur entre une troisième tension négative et la masse, cette troisième tension négative étant celle qui est reliée au côté inférieur du circuit de décharge.

Des variantes de l'invention dérivent ces trois tensions négatives de différentes manières, directement ou indirectement, à partir de la pile.

L'invention a pour objet un défibrillateur/ stimulateur cardiaque implantable à générateur de chocs multiphasiques comprenant:
- une pile pour fournir l'énergie,
- des circuits de commande pour déclencher la stimulation et les chocs,
- des circuits de stimulation pour fournir des impulsions de stimulation au coeur,
- un circuit de charge de choc pour convertir l'énergie de la pile en énergie de choc,
- un condensateur pour stocker l'énergie de choc,
- deux commutateurs électroniques sur le côté supérieur et deux commutateurs électroniques sur le côté inférieur d'une configuration en pont en H pour relier ledit condensateur à une charge avec une polarité sélective,
- deux dispositifs de commande isolés du côté supérieur pour actionner sélectivement chaque commutateur du côté supérieur en réponse à un signal correspondant provenant desdits circuits de commande,
- deux dispositifs de commande du côté inférieur pour actionner sélectivement chaque commutateur du côté inférieur en réponse à un signal correspondant en provenance desdits circuits de commande, et
- un circuit fournissant de la puissance en provenance de ladite pile auxdits circuits de commande, circuits de stimulation/détection et dispositif de commande du côté inférieur,
caractérisé en ce qu'il comporte :
- une connexion entre la borne positive de ladite pile et la masse,
- des moyens pour dériver une première tension d'alimentation négative pour alimenter lesdits circuits de commande entre ladite première tension d'alimentation négative et la masse,
- des moyens pour dériver une seconde tension d'alimentation négative pour alimenter lesdits circuits de stimulation entre ladite seconde tension d'alimentation négative et la masse,
- des moyens pour dériver une troisième tension d'alimentation négative pour alimenter lesdits dispositifs de commande du côté inférieur entre ladite troisième tension d'alimentation négative et la masse,
- une connexion entre ladite troisième tension d'alimentation négative et la borne négative dudit condensateur et du circuit d'application de chocs à pont en H.

Avantageusement :
- les moyens pour dériver une première tension d'alimentation négative pour alimenter lesdits circuits de commande comprennent une connexion directe avec la borne négative de la pile ;
- les moyens pour dériver une première tension d'alimentation négative pour alimenter lesdits circuits de commande comprennent un circuit régulateur de tension pour stabiliser la tension de la pile ;
- la première tension d'alimentation négative est comprise entre approximativement -1,0 et -3,0 V ;
- les moyens pour dériver une seconde tension d'alimentation négative pour alimenter lesdits circuits de stimulation et de détection comprennent une connexion directe avec la borne négative de la pile ;
- les moyens pour dériver une seconde tension d'alimentation négative pour alimenter lesdits circuits de stimulation comprennent une connexion directe avec ladite première tension d'alimentation négative ;
- les moyens pour dériver une seconde tension d'alimentation négative pour alimenter lesdits circuits de stimulation comprennent un circuit régulateur de tension pour stabiliser la tension de la pile;
- les moyens pour dériver une seconde tension d'alimentation négative pour alimenter lesdits moyens de stimulation et de détection comprennent un circuit multiplicateur de tension fonctionnant à partir de ladite première tension d'alimentation négative ;
- la seconde tension d'alimentation négative est comprise entre approximativement -0,5 et -10 V ;
- la seconde tension d'alimentation négative est programmable ;
- les moyens pour dériver une troisième tension d'alimentation négative pour alimenter lesdits dispositifs de commande du côté inférieur comprennent une connexion directe avec la borne négative de la pile ;
- les moyens pour dériver une troisième tension d'alimentation négative pour alimenter lesdits dispositifs de commande du côté inférieur comprennent une connexion directe avec ladite première tension d'alimentation négative ;
- les moyens pour dériver une troisième tension d'alimentation négative pour alimenter lesdits dispositifs de commande du côté inférieur comprennent un circuit régulateur de tension pour stabiliser la tension de la pile;
- les moyens pour dériver une troisième tension d'alimentation négative pour alimenter lesdits dispositifs de commande du côté inférieur comprennent un circuit multiplicateur de tension fonctionnant à partir de ladite première tension d'alimentation négative ;
- la troisième tension d'alimentation négative est comprise entre approximativement -10 et -15 V ;
- le défibrillateur/stimulateur comporte une résistance entre la troisième tension d'alimentation négative et la borne négative du circuit d'application de chocs à pont en H ;
- la résistance a une valeur approximativement de 0,03 ohm.

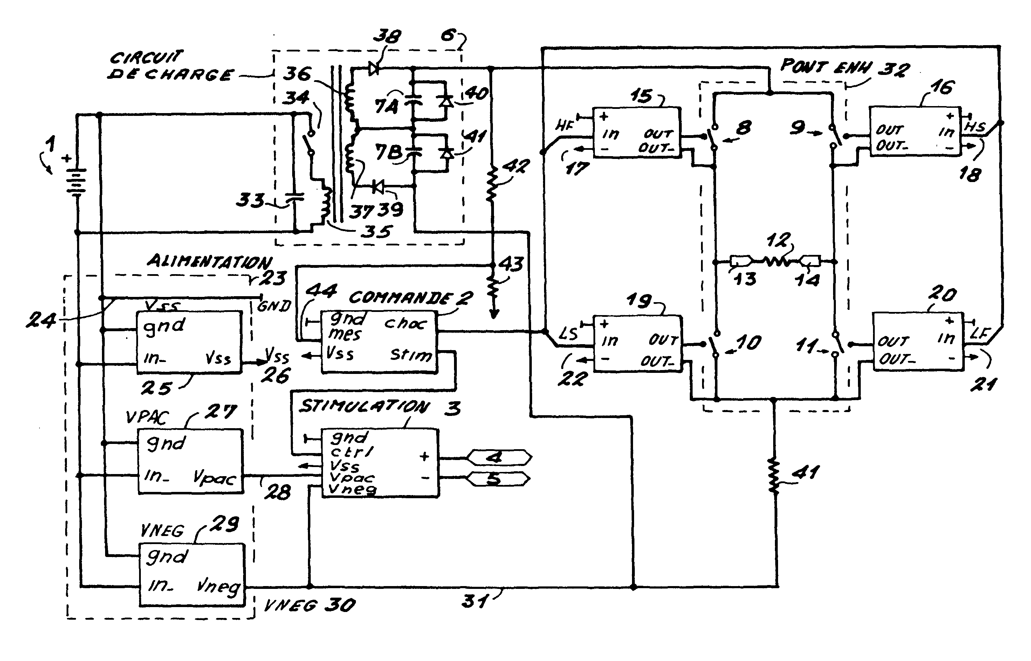

La figure 1 est une vue d'ensemble de l'invention appliquée à un défibrillateur/stimulateur cardiaque implantable.

En se référant à la figure 1, la pile montrée en 1 fournit l'énergie à tous les circuits du dispositif. Les circuits de commande en 2 déterminent les temps de la stimulation et des chocs. Les circuits de stimulation en 3 fournissent les impulsions de stimulation, aux bornes 4 et 5 des électrodes. La figure montre en 6 un circuit de charge de chocs pour convertir l'énergie de la pile en une énergie de choc, qui comprend des condensateurs pour stocker l'énergie de choc en 7A et 7B.

La figure 1 montre également deux commutateurs électroniques 8 et 9 du côté supérieur, et deux commutateurs électroniques 10 et 11 du côté inférieur de la configuration à pont en H 32 pour relier ledit condensateur, avec une polarité déterminable, à une charge 12 placée entre les bornes 13 et 14 des électrodes de choc. La figure montre deux dispositifs de commande isolés 15 et 16 du côté supérieur pour actionner sélectivement chaque commutateur du côté supérieur en réponse à un signal correspondant HF en 17 et HS en 18 en provenance desdits circuits de commande 2.

Toujours en se référant à la figure 1, celle-ci montre deux dispositifs de commande 19 et 20 du côté inférieur pour actionner sélectivement chaque commutateur du côté inférieur en réponse à des signaux correspondants LF en 21 et LS en 22 en provenance desdits circuits de commande 2.

La figure montre également un circuit 23 fournissant la puissance à partir de la pile 1 aux circuits de commande 2, aux circuits de stimulation 3 et aux dispositifs de commande 19 et 20 du côté inférieur. L'alimentation 23 comprend:
- une connexion 24 entre la borne positive de la pile 1 et la masse;
- une alimentation en VSS 25 pour dériver une première tension d'alimentation négative appelée VSS en 26 pour alimenter les circuits de commande 2 entre VSS et la masse, pour servir de niveau de référence logique dans l'ensemble du dispositif, et pour alimenter les dispositifs de commande 15 et 16 du côté supérieur,
- une alimentation en VPAC 27 pour dériver une seconde tension d'alimentation négative appelée VPAC en 28 pour alimenter les circuits de stimulation 3 entre VPAC et la masse,
- une alimentation en VNEG 29 pour dériver une troisième tension d'alimentation négative appelée VNEG en 30 pour alimenter les dispositifs de commande 19 et 20 du côté inférieur entre VNEG et la masse,
- une connexion 31 entre VNEG et la borne négative des condensateurs 7A, 7B, et avec le circuit d'application de chocs à pont en H 32 par l'intermédiaire de la résistance optionnelle de détection 41. La valeur de la résistance 41 est très faible, approximativement de 0,03 ohm, et la tension est essentiellement de zéro à ses bornes pendant le fonctionnement normal du circuit.

Dans l'alimentation 23:
* VSS en 26 est comprise entre approximativement -1,0 et -3,0 V, pour alimenter le circuit de commande et les dispositifs de commande du côté supérieur, et pour servir de niveau bas de référence logique.
* VPAC en 28 est comprise entre approximativement -0,5 et -10 V, éventuellement programmable, pour alimenter l'impulsion de stimulation;
* VNEG en 30 est comprise entre approximativement -10 et -15 V, pour alimenter les dispositifs de commande des commutateurs du côté inférieur dans le générateur de chocs.

De nombreuses autres configurations existent dans les limites du champ d'application de l'invention pour générer ces trois tensions d'alimentation négatives. La figure 1 montre l'une de ces configurations qui comprend des alimentations séparées régulées 25, 27 et 29 fonctionnant directement à partir de la pile. Des exemples d'autres configurations comprennent: (1) la connexion d'une ou plusieurs des sorties d'alimentation directement à la borne négative de la pile sans régulation, (2) la connexion d'une ou plusieurs des sorties d'alimentation à un unique régulateur, ou (3) la dérivation des seconde et troisième tensions à partir de la première tension régulée en utilisant des multiplicateurs de tension.

En se référant toujours à la figure 1, les circuits de stimulation 3 comprennent une logique fonctionnant entre la masse et VSS, et une génération de signaux de stimulation fonctionnant entre la masse et VPAC. Les circuits 3 peuvent également utiliser VNEG qui est toujours au moins autant négative que VPAC, pour fournir une région de fonctionnement en mode commun comprenant VPAC.

Toujours à la figure 1, le circuit de charge en 6 convertit la tension de la pile en une haute tension pour les chocs. L'entrée du circuit de charge est reliée aux bornes de la pile 1, découplée par le condensateur 33. La sortie du circuit de charge est reliée aux bornes du pont en H 32. Très brièvement, quand le commutateur 34 se ferme, le courant monte dans le primaire 35 du transformateur. Quand le commutateur 34 s'ouvre, le courant commence à passer dans les secondaires 36 et 37, chargeant les condensateurs de choc 7A et 7B par l'intermédiaire des diodes 38 et 39. Dans l'illustration des principes de l'invention de la figure 1, deux condensateurs sont montrés en série, bien que de nombreuses autres configurations ne comportant qu'un unique condensateur ou d'autres combinaisons série et parallèle puissent être également utilisées tout en restant dans le champ d'application de l'invention. Le circuit de charge charge le condensateur 7 à une tension préétablie déterminée par des moyens de régulation qui ne sont pas représentés ici mais qui sont montrés dans de nombreux exemples de l'art antérieur.

Quand les circuits de commande 2 appliquent HF en 17 et LF en 21, le dispositif de commande 15 du côté supérieur et le dispositif de commande 20 du côté inférieur acheminent ces signaux de commande de façon à fermer les commutateurs 8 et 11 respectivement, de manière que le courant s'écoule depuis le condensateur 7 à travers la charge 12 dans un premier sens, de 13 à 14 (ceci constituant la première phase du choc). Ensuite les circuits 2 de commande à basse tension annihilent les signaux acheminés sur toutes les sorties de commande 17, 18, 21, 22, de façon à ouvrir tous les commutateurs. Ceci détermine un délai entre les phases, donnant le temps à tous les commutateurs de s'ouvrir. Ensuite, les circuits basse tension 2 appliquent HS en 18 et LS en 22, de sorte que le dispositif de commande 16 du côté supérieur et le dispositif de commande 19 du côté inférieur ferment les commutateurs 9 et 10 respectivement, de façon que le courant passe du condensateur à travers la charge 12 dans un second sens, de 14 à 13 (ceci constituant la seconde phase du choc). Ensuite, les circuits basse tension annihilent les signaux acheminés sur toutes les sorties de commande 17, 18, 21 et 22, de façon à ouvrir tous les commutateurs et tronquer la seconde phase. Les circuits basse tension peuvent optionnellement poursuivre cette séquence pour générer des phases additionnelles.

Les commutateurs 8 à 11 peuvent être constitués par des MOSFET, des IGBT ou des SCR ainsi que cela est connu des concepteurs qualifiés. Comme les SCR ne peuvent pas être ouverts tant que le courant qui les traverse n'est pas tombé à zéro, ils ne peuvent être utilisés que dans le commutateur du côté supérieur ou dans la commutateur du côté inférieur pour une phase, mais non pour les deux côtés dans la même phase. Les MOSFET ou les IGBT doivent avoir des diodes en série comme montré dans l'art antérieur, pour éviter qu'une défibrillation externe ne soit conduite dans le sens opposé à travers les commutateurs.

Les dispositifs de commande de commutateurs 19 et 20 du côté inférieur traduisent un signal de niveau logique entre VSS et GND en un signal plus négatif entre VNEG et GND. Des exemples sont montrés dans l'art antérieur cité plus haut. Les dispositifs de commande de commutateurs 15 et 16 du côté supérieur traduisent un signal de niveau logique entre VSS et GND pour fournir une sortie isolée de signal de commande. Des exemples utilisant des transformateurs d'impulsions pour des commutateurs SCR, ou des transformateurs RF pour des commutateurs MOSFET ou IGBT, sont montrés dans l'art antérieur cité plus haut.

La résistance 41 montre un avantage du circuit de l'invention par comparaison avec des circuits à sortie isolée. Pendant un choc, la tension aux bornes de la résistance 41 dépend directement du courant de choc. Quand le courant de choc passe, le côté de la résistance 41 qui est relié aux dispositifs de commande 19 et 20 du côté inférieur devient positif par rapport à VNEG. Ainsi, les circuits (non montrés) qui fonctionnent entre VDD et VNEG pourraient être utilisés pour mesurer le courant de choc pendant un choc.

Il peut être utile d'inclure des moyens pour mesurer la sortie haute tension du circuit de charge 6. Le diviseur de tension consistant en les résistances 42 et 43 est utilisé dans l'exemple représenté pour échelonner la sortie haute tension du circuit de charge par rapport à une basse rapportée à VSS. Si le rapport de division est sélectionné correctement, à 1000:1 par exemple, la tension à la jonction 44 des résistances 42 et 43 reste alors entre VDD et VSS, ce qui convient pour que les circuits de commande 2 effectuent une mesure.

Un autre avantage apporté par le raccordement du circuit d'application de chocs à une alimentation négative VNEG plutôt qu'à la masse devient évident quand on considère que: (a) le circuit de charge 6 se charge et maintient la tension aux bornes du condensateur 7 à une valeur constante comme expliqué précédemment; (b) l'alimentation en VSS 25 charge VSS à tension constante; et (c) le courant passant par les résistances 42 et 43 a tendance à tirer le point haut du condensateur 7 vers la masse quand il est chargé. Comme le condensateur 7 est sous tension constante, ceci pousse le point bas du condensateur 7 pour le rendre encore plus négatif, fournissant des moyens additionnels pour charger l'alimentation négative VNEG quand le condensateur 7 est chargé. Lorsque le condensateur 7 est chargé, ceci signifie que le défibrillateur est armé pour envoyer un choc. A ce moment, il est important qu'il y ait une forte différence de tension entre VDD et VNEG pour fournir une tension maximale de commande de gâchette pour les dispositifs de commande 19 et 20 du côté inférieur. Le fait de rapporter le circuit de chocs à une alimentation négative, fournit un courant de charge additionnel pour cette alimentation négative, juste au moment où il est le plus nécessaire.

## Revendications

1. Défibrillateur/stimulateur cardiaque implantable à générateur de chocs multiphasiques comprenant:
- une pile (1) pour fournir l'énergie,
- des circuits (2) de commande pour déclencher la stimulation et les chocs,
- des circuits (3) de stimulation pour fournir des impulsions de stimulation au coeur,
- un circuit (6) de charge de choc pour convertir l'énergie de la pile en énergie de choc,
- un condensateur (7) pour stocker l'énergie de choc,
- deux commutateurs électroniques (8, 9) sur le côté supérieur et deux commutateurs électroniques (10, 11) sur le côté inférieur d'une configuration en pont en H (32) pour relier ledit condensateur (7) à une charge (12) avec une polarité sélective,
- deux dispositifs (15, 16) de commande isolés du côté supérieur pour actionner sélectivement chaque commutateur (8, 9) du côté supérieur en réponse à un signal correspondant provenant desdits circuits (2) de commande,
- deux dispositifs (19, 20) de commande du côté inférieur pour actionner sélectivement chaque commutateur (10, 11) du côté inférieur en réponse à un signal correspondant en provenance desdits circuits de commande (2), et
- un circuit (23) fournissant de la puissance en provenance de ladite pile (1) auxdits circuits (2) de commande, circuits (3) de stimulation/détection et dispositifs (19, 20) de commande du côté inférieur,
**caractérisé en ce qu'**il comporte :
- une connexion (24) entre la borne positive de ladite pile (1) et la masse,
- des moyens (25) pour dériver une première tension d'alimentation négative (VSS) pour alimenter lesdits circuits (2) de commande entre ladite première tension d'alimentation négative (VSS) et la masse,
- des moyens (27) pour dériver une seconde tension d'alimentation négative (VPAC) pour alimenter lesdits circuits (3) de stimulation entre ladite seconde tension d'alimentation négative (VPAC) et la masse,
- des moyens (29) pour dériver une troisième tension d'alimentation négative (VNEG) pour alimenter lesdits dispositifs (19, 20) de commande du côté inférieur entre ladite troisième tension d'alimentation négative (VNEG) et la masse,
- une connexion (31) entre ladite troisième tension d'alimentation négative (VNEG) et la borne négative dudit condensateur (7) et du circuit d'application de chocs à pont en H (32).

2. Défibrillateur/stimulateur cardiaque selon la revendication 1, **caractérisé en ce que** lesdits moyens (25) pour dériver une première tension d'alimentation négative (VSS) pour alimenter lesdits circuits (2) de commande comprennent une connexion directe avec la borne négative de la pile (1).

3. Défibrillateur/stimulateur cardiaque selon la revendication 1, **caractérisé en ce que** lesdits moyens (25) pour dériver une première tension d'alimentation négative (VSS) pour alimenter lesdits circuits (2) de commande comprennent un circuit régulateur de tension pour stabiliser la tension de la pile (1).

4. Défibrillateur/stimulateur cardiaque selon la revendication 3, **caractérisé en ce que** ladite première tension d'alimentation négative est comprise entre approximativement -1,0 et -3,0 V.

5. Défibrillateur/stimulateur cardiaque selon l'une des revendications 1 à 4, **caractérisé en ce que** lesdits moyens (27) pour dériver une seconde tension d'alimentation négative (VPAC) pour alimenter lesdits circuits (3) de stimulation et de détection comprennent une connexion directe avec la borne négative de la pile (1).

6. Défibrillateur/stimulateur cardiaque selon l'une des revendications 1 à 4, **caractérisé en ce que** lesdits moyens (27) pour dériver une seconde tension d'alimentation négative (VPAC) pour alimenter lesdits circuits (3) de stimulation comprennent une connexion directe avec ladite première tension d'alimentation négative (VSS).

7. Défibrillateur/stimulateur cardiaque selon l'une des revendications 1 à 4, **caractérisé en ce que** lesdits moyens (27) pour dériver une seconde tension d'alimentation négative (VPAC) pour alimenter lesdits circuits (3) de stimulation comprennent un circuit régulateur de tension pour stabiliser la tension de la pile (1).

8. Défibrillateur/stimulateur cardiaque selon l'une des revendications 1 à 4, **caractérisé en ce que** lesdits moyens (27) pour dériver une seconde tension d'alimentation négative (VPAC) pour alimenter lesdits moyens (3) de stimulation et de détection comprennent un circuit multiplicateur de tension fonctionnant à partir de ladite première tension d'alimentation négative (VSS).

9. Défibrillateur/stimulateur cardiaque selon l'une des revendications 7 ou 8, **caractérisé en ce que** ladite seconde tension d'alimentation négative (VPAC) est comprise entre approximativement -0,5 et -10 V.

10. Défibrillateur/stimulateur cardiaque selon la revendication 9, **caractérisé en ce que** ladite seconde tension d'alimentation négative (VPAC) est programmable.

11. Défibrillateur/stimulateur cardiaque selon l'une des revendications 1 à 10, **caractérisé en ce que** lesdits moyens (29) pour dériver une troisième tension d'alimentation négative (VNEG) pour alimenter lesdits dispositifs (19, 20) de commande du côté inférieur comprennent une connexion directe avec la borne négative de la pile (1).

12. Défibrillateur/stimulateur cardiaque selon l'une des revendications 1 à 10, **caractérisé en ce que** lesdits moyens (29) pour dériver une troisième tension d'alimentation négative (VNEG) pour alimenter lesdits dispositifs (19, 20) de commande du côté inférieur comprennent une connexion directe avec ladite première tension d'alimentation négative (VSS).

13. Défibrillateur/stimulateur cardiaque selon l'une revendications 1 à 10, **caractérisé en ce que** lesdits moyens (29) pour dériver une troisième tension d'alimentation négative (VNEG) pour alimenter lesdits dispositifs (19, 20) de commande du côté inférieur comprennent un circuit régulateur de tension pour stabiliser la tension de la pile (1).

14. Défibrillateur/stimulateur cardiaque selon l'une revendications 1 à 10, **caractérisé en ce que** lesdits moyens (29) pour dériver une troisième tension d'alimentation négative (VNEG) pour alimenter lesdits dispositifs (19, 20) de commande du côté inférieur comprennent un circuit multiplicateur de tension fonctionnant à partir de ladite première tension d'alimentation négative (VSS).

15. Défibrillateur/stimulateur cardiaque selon l'une des revendications 13 ou 14, **caractérisé en ce que** ladite troisième tension d'alimentation négative (VNEG) est comprise entre approximativement -10 et -15 V.

16. Défibrillateur/stimulateur cardiaque selon l'une des revendications 1 à 15, **caractérisé en ce qu'**il comporte une résistance (41) entre ladite troisième tension d'alimentation négative (VNEG) et la borne négative du circuit d'application de chocs à pont en H (32).

17. Défibrillateur/stimulateur cardiaque selon la revendication 16, **caractérisé en ce que** ladite résistance (41) a une valeur approximativement de 0,03 ohm.

## Claims

1. An implantable defibrillator/cardiac pacemaker having a multiphase shock generator, comprising:
- a battery (1) for supplying energy,
- control circuits (2) for triggering pacing and shocks,
- pacing circuits (3) for supplying pacing pulses to the heart,
- a shock charging circuit (6) for converting the energy of the battery into shock energy,
- a capacitor (7) for storing the shock energy,
- two electronic commutators (8, 9) on the upper side and two electronic commutators (10, 11) on the lower side of an H-shaped bridge configuration (32) for connecting said capacitor (7) to a load (12) with a selective polarity,
- two isolated control devices (15, 16) of the upper side for selectively actuating each commutator (8, 9) of the upper side in response to a corresponding signal coming from said control circuits (2),
- two control devices (19, 20) of the lower side for selectively actuating each commutator (10, 11) of the lower side in response to a corresponding signal coming from said control circuits (2), and
- a circuit (23) providing power from said battery (1) to said control circuits (2), pacing/detection circuits (3) and control devices (19, 20) of the lower side,
**characterised in that** it comprises:
- a connection (24) between the position terminal of said battery (1) and the earth,
- means (25) for branching off a first negative supply voltage (VSS) for supplying said control circuits (2) between said first negative supply voltage (VSS) and the earth,
- means (27) for branching off a second negative supply voltage (VPAC) for supplying said pacing circuits (3) between said second negative supply voltage (VPAC) and the earth,
- means (29) for branching off a third negative supply voltage (VNEG) for supplying said control devices (19, 20) of the lower side between said third negative supply voltage (VNEG) and the earth,
- a connection (31) between said third negative supply voltage (VNEG) and the negative terminal of said capacitor (7) and of the H-shaped bridge circuit (32) for applying shocks.

2. A defibrillator/cardiac pacemaker according to Claim 1, **characterised in that** said means (25) for branching off a first negative supply voltage (VSS) for supplying said control circuits (2) comprise a direct connection to the negative terminal of the battery (1).

3. A defibrillator/cardiac pacemaker according to Claim 1, **characterised in that** said means (25) for branching off a first negative supply voltage (VSS) for supplying said control circuits (2) comprise a voltage regulation circuit for stabilising the voltage of the battery (1).

4. A defibrillator/cardiac pacemaker according to Claim 3, **characterised in that** said first negative supply voltage lies between approximately -1.0 and -3.0 V.

5. A defibrillator/cardiac pacemaker according to one of Claims 1 to 4, **characterised in that** said means (27) for branching off a second negative supply voltage (VPAC) for supplying said pacing and detection circuits (3) comprise a direct connection to the negative terminal of the battery (1).

6. A defibrillator/cardiac pacemaker according to one of Claims 1 to 4, **characterised in that** said means (27) for branching off a second negative supply voltage (VPAC) for supplying said pacing circuits (3) comprise a direct connection to said first negative supply voltage (VSS).

7. A defibrillator/cardiac pacemaker according to one of Claims 1 to 4, **characterised in that** said means (27) for branching off a second negative supply voltage (VPAC) for supplying said pacing circuits (3) comprise a voltage regulation circuit for stabilising the voltage of the battery (1).

8. A defibrillator/cardiac pacemaker according to one of Claims 1 to 4, **characterised in that** said means (27) for branching off a second negative supply voltage (VPAC) for supplying said pacing and detection circuits (3) comprise a voltage multiplier circuit which operates from said first negative supply voltage (VSS).

9. A defibrillator/cardiac pacemaker according to one of Claims 7 or 8, **characterised in that** said second negative supply voltage (VPAC) lies between approximately -0.5 and -10 V.

10. A defibrillator/cardiac pacemaker according to Claim 9, **characterised in that** said second negative supply voltage (VPAC) is programmable.

11. A defibrillator/cardiac pacemaker according to one of Claims 1 to 10, **characterised in that** said means (29) for branching off a third negative supply voltage (VNEG) for supplying said control devices (19, 20 ) of the lower side comprise a direct connection to the negative terminal of the battery (1).

12. A defibrillator/cardiac pacemaker according to one of Claims 1 to 10, **characterised in that** said means (29) for branching off a third negative supply voltage (VNEG) for supplying said control devices (19, 20) comprise a direct connection to said first negative supply voltage (VSS).

13. A defibrillator/cardiac pacemaker according to one of Claims 1 to 10, **characterised in that** said means (29) for branching off a third negative supply voltage (VNEG) for supplying said control devices (19, 20) of the lower side comprise a voltage regulation circuit for stabilising the voltage of the battery (1).

14. A defibrillator/cardiac pacemaker according to one of Claims 1 to 10, **characterised in that** said means (29) for branching off a third negative supply voltage (VNEG) for supplying said control devices (19, 20) of the lower side comprise a voltage multiplier circuit which operates from said first negative supply voltage (VSS).

15. A defibrillator/cardiac pacemaker according to one of Claims 13 or 14, **characterised in that** said third negative supply voltage (VNEG) lies between approximately -10 and -15 V.

16. A defibrlllator/cardiac pacemaker according to one of Claims 1 to 15, **characterised in that** it comprises a resistor (41) between said third negative supply voltage (VNEG) and the negative terminal of the H-shaped bridge circuit (32) for applying shocks.

17. A defibrillator/cardiac pacemaker according to Claim 16, **characterised in that** said resistor (41) has a value of approximately 0.03 ohm.

## Patentansprüche

1. Implantierbarer Defibrillator/Herzschrittmacher mit mehrphasigem Impulsgenerator, welcher aufweist:
eine Batterie (1) zur Energieversorgung,
Steuerschaltungen (2) zur Auslösung einer Stimulation und von Impulsen,
Stimulationsschaltungen (3) zur Zufuhr von Herzstimulationsimpulsen,
eine Impulsladungsschaltung (6) zur Umwandlung der Energie der Batterie in Impulsenergie,
einen Kondensator (7) zur Speicherung der Impulsenergie,
zwei elektronische Schalter (8, 9) auf der Oberseite und zwei elektronische Schalter (10, 11) auf der Unterseite in einer H-Brückenkonfiguration (32), um den Kondensator (7) an eine Ladung (12) mit einer selektiven Polarität anzuschließen,
zwei isolierte Steuervorrichtungen (15, 16) auf der Oberseite zur wahlweisen Betätigung jedes Schalters (8, 9) der Oberseite in Reaktion auf ein von den Steuerschaltungen (2) kommendes, entsprechendes Signal,
zwei Steuervorrichtungen (19, 20) auf der Unterseite zur wahlweisen Betätigung jedes Schalters (10, 11) der Unterseite in Reaktion auf ein von den Steuerschaltungen (2) kommendes, entsprechendes Signal und
eine Schaltung (23), welche Energie von Seiten der Batterie (1) den Steuerschaltungen (2), Stimuliations-/ Erfassungsschaltungen (3) und Steuervorrichtungen (19, 20) der Unterseite zuführt,
**dadurch gekennzeichnet, dass** er aufweist:
eine Verbindung (24) zwischen dem positiven Anschluss der Batterie (1) und Masse,
eine Einrichtung (25) zur Ableitung einer ersten negativen Speisespannung (VSS), um die Steuerschaltungen (2) zwischen der ersten negativen Speisespannung (VSS) und Masse zu versorgen,
eine Einrichtung (27) zur Ableitung einer zweiten negativen Speisespannung (VPAC), um die Stimulationsschaltungen (3) zwischen der zweiten negativen Speisespannung (VPAC) und Masse zu versorgen,
eine Einrichtung (29) zur Ableitung einer dritten negativen Speisespannung (VNEG), um die Steuervorrichtungen (19,20) der Unterseite zwischen der dritten negativen Speisespannung (VNEG) und Masse zu versorgen,
eine Verbindung (31) zwischen der dritten negativen Speisespannung (VNEG) und dem negativen Anschluss des Kondensators (7) und der Impulsanwendungs-H-Brückenschaltung (32).

2. Defibrillator/Herzschrittmacher nach Anspruch 1, **dadurch gekennzeichnet, dass** die Einrichtung (25) zur Ableitung einer ersten negativen Speisespannung (VSS) zur Versorgung der Steuerschaltungen (2) eine direkte Verbindung mit dem negativen Anschluss der Batterie (1) aufweist.

3. Defibrillator/Herzschrittmacher nach Anspruch 1, **dadurch gekennzeichnet, dass** die Einrichtung (25) zur Ableitung einer ersten negativen Speisespannung (VSS), zur Versorgung der Steuerschaltungen (2) eine Spannungsregulierungsschaltung zur Stabilisierung der Spannung der Batterie (1) aufweist.

4. Defibrillator/Herzschrittmacher nach Anspruch 3, **dadurch gekennzeichnet, dass** die erste negative Speisespannung ungefähr zwischen -1,0 V und -3,0 V liegt.

5. Defibrillator/Herzschrittmacher nach einem der Ansprüche 1 bis 4, **dadurch gekennzeichnet, dass** die Einrichtung (27) zur Ableitung einer zweiten negativen Speisespannung (VPAC) zur Versorgung der Stimulations- und Erfassungsschaltungen (3) eine direkte Verbindung mit dem negativen Anschluss der Batterie (1) aufweist.

6. Defibrillator/Herzschrittmacher nach einem der Ansprüche 1 bis 4, **dadurch gekennzeichnet, dass** die Einrichtung (27) zur Ableitung einer zweiten negativen Speisespannung (VPAC) zur Versorgung der Stimulationsschaltungen (3) eine direkte Verbindung mit der ersten negativen Speisespannung (VSS) aufweist.

7. Defibrillator/Herzschrittmacher nach einem der Ansprüche 1 bis 4, **dadurch gekennzeichnet, dass** die Einrichtung (27) zur Ableitung einer zweiten negativen Speisespannung (VPAC) zur Versorgung der Stimulationsschaltungen (3) eine Spannungsregulierungsschaltung zur Stabilisierung der Spannung der Batterie (1) aufweist.

8. Defibrillator/Herzschrittmacher nach einem der Ansprüche 1 bis 4, **dadurch gekennzeichnet, dass** die Einrichtung (27) zur Ableitung einer zweiten negativen Speisespannung (VPAC) zur Versorgung der Stimulations- und Erfassungsschaltungen (3) eine Spannungs-Multiplikationsschaltung aufweist, welche ausgehend von der ersten negativen Speisespannung (VSS) funktioniert.

9. Defibrillator/Herzschrittmacher nach einem der Ansprüche 7 oder 8, **dadurch gekennzeichnet, dass** die zweite negative Speisespannung (VPAC) ungefähr zwischen -0,5 V und -10 V liegt.

10. Defibrillator/Herzschrittmacher nach Anspruch 9, **dadurch gekennzeichnet, dass** die zweite negative Speisespannung (VPAC) programmierbar ist.

11. Defibrilator/Herzschrittmacher nach einem der Ansprüche 1 bis 10, **dadurch gekennzeichnet, dass** die Einrichtung (29) zur Ableitung einer dritten negativen Speisespannung (VNEG) zur Versorgung der Steuervorrichtungen (19, 20) der Unterseite eine direkte Verbindung mit dem negativen Anschluss der Batterie (1) aufweist.

12. Defibrillator/Herzschrittmacher nach einem der Ansprüche 1 bis 10, **dadurch gekennzeichnet, dass** die Einrichtung (29) zur Ableitung einer dritten negativen Speisespannung (VNEG) zur Versorgung der Steuervorrichtungen (19, 20) der Unterseite eine direkte Verbindung mit der ersten negativen Speisespannung (VSS) aufweist.

13. Defibrillator/Herzschrittmacher nach einem der Ansprüche 1 bis 10, **dadurch gekennzeichnet, dass** die Einrichtung (29) zur Ableitung einer dritten negativen Speisespannung (VNEG) zur Versorgung der Steuervorrichtungen (19, 20) der Unterseite eine Spannungsregulierungsschaltung zur Stabilisierung der Spannung der Batterie (1) aufweist.

14. Defibrillator/Herzschrittmacher nach einem der Ansprüche 1 bis 10, **dadurch gekennzeichnet, dass** die Einrichtung (29) zur Ableitung einer dritten negativen Speisespannung (VNEG) zur Versorgung der Steuervorrichtungen (19, 20) der Unterseite eine Spannungs-Multiplikationsschaltung aufweist, welche ausgehend von der ersten negativen Speisespannung (VSS) funktioniert.

15. Defibrillator/Herzschrittmacher nach einem der Ansprüche 13 oder 14, **dadurch gekennzeichnet, dass** die dritte negative Speisespannung (VNEG) ungefähr zwischen -10 V und -15 V liegt.

16. Defibrillator/Herzschrittmacher nach einem der Ansprüche 1 bis 15, **dadurch gekennzeichnet, dass** er einen Widerstand (41) zwischen der dritten negativen Speisespannung (VNEG) und dem negativen Anschluss der Impulsanwendungs-H-Brückenschaltung (32) aufweist.

17. Defibrillator/Herzschrittmacher nach Anspruch 16, **dadurch gekennzeichnet, dass** der Widerstand (41) einen Wert von ungefähr 0,03 Ohm aufweist.
